# EUROPÄISCHE PATENTSCHRIFT

(11) **EP 0 734 285 B1**
(45) Veröffentlichungstag und Bekanntmachung des Hinweises auf die Patenterteilung: **03.06.1998**
(21) Anmeldenummer: 95904439.7
(22) Anmeldetag: 13.12.1994
(51) Int. Cl.: B01D 39/04, A61K 31/79, A61K 33/18, A61K 33/40

(54) **TIEFENFILTER ZUR ABTÖTUNG VON MIKROORGANISMEN UND INAKTIVIERUNG VON VIREN UND DESSEN ANWENDUNG**
DEEP-BED FILTER FOR EXTERMINATING MICRO-ORGANISMS AND INACTIVATING VIRUSES AND ITS USE
FILTRE A LIT PROFOND POUR L'EXTERMINATION DE MICRO-ORGANISMES ET L'INACTIVATION DE VIRUS, ET SON UTILISATION

(30) Priorität: 17.12.1993 DE 4343226
(43) Veröffentlichungstag der Anmeldung: 02.10.1996
(73) Patentinhaber: BASF AKTIENGESELLSCHAFT, 67056 Ludwigshafen (DE); SCHENK FILTERBAU GMBH, 73550 Waldstetten (DE)
(72) Erfinder: BREITENBACH, Jörg, D-68199 Mannheim (DE); FUSSNEGGER, Bernhard, D-67489 Kirrweiler (DE); LANG, Siegfried, D-67071 Ludwigshafen (DE)
(86) Internationale Anmeldenummer: EP9404092
(87) Internationale Veröffentlichungsnummer: WO9516511

(56) Entgegenhaltungen:
- WO-A-85/02422
- WO-A-93/06911
- WO-A-94/00161
- US-A- 3 216 579

## Beschreibung

Die vorliegende Erfindung betrifft als biocide Tiefenfilter geeignete Formkörper aus Fasermaterialien und darin eingebetteten Partikeln aus Crospovidone-Jod. Weiterhin betrifft die Erfindung Schichtenfilter enthaltenden mindestens einen der Formkörper als Tiefenfilterschicht. Die Erfindung betrifft auch die Verwendung der Formkörper und der Schichtenfilter zur Abtötung von Mikroorganismen oder Inaktivierung von Viren für solche enthaltenden Flüssigkeiten.

Zum Reinigen von Wasser, Blut oder Blutpräparaten, aber auch von anderen Körperflüssigkeiten oder Injektionslösungen werden diese Produkte filtriert. Will man gleichzeitig mit der Reinigung auch eine Abtötung von pathogenen Keimen erzielen, so muß man den Filtrier- und Reinigungsvorgang mit Maßnahmen kombinieren, die auch eine entsprechende Inaktivierung bzw. Abtötung von pathogenen Keimen bewirken. Hierfür sind schon verschiedene Verfahren und Vorrichtungen bekannt. So ist es aus der WO -92/040 31 bekannt, daß man Blut, Blutderivate und andere Körperflüssigkeiten mit löslichen PVP-Jod-Präparaten (Povidone- Jod) durch Abtöten von pathogenen Keimen reinigen kann, ohne daß die zu reinigenden Substanzen dabei geschädigt werden. Zu diesem Zweck wird die zu reinigende Flüssigkeit mit dem Povidone-Jod behandelt und die so behandelte Flüssigkeit wird dann über Filter geleitet. Die abgetöteten Keime und andere Verunreinigungen verbleiben dann im Filterkuchen, wahrend die ablaufende Flüssigkeit die gereinigten Produkte enthält. Obwohl ein solches Verfahren durchaus wirksam ist, ist es ein mehrstufiges Verfahren, bei dem man zunächst das gelöste Povidone-Jod mit der zu reinigenden Flüssigkeit zusammenbringt und anschließend dann filtriert. Nachteilig an diesem Verfahren ist, daß das gelöste Povidone dabei (selbst nach einer Abtrennung des Jods) im Filtrat gelöst und daraus praktisch nicht wieder abgetrennt werden kann. Das hat zur Folge, daß ein solches Verfahrensprodukt nicht mehr direkt appliziert werden kann, da es das nicht nierengängige Povidone noch enthält.

Aus der DE-41 19 288 ist es weiterhin bekannt, pharmazeutische oder biologische und andere Flüssigkeiten durch Schichtenfiltration zu reinigen. Da solche Flüssigkeiten nach dem Reinigungsvorgang frei von Fremdbestandteilen, insbesondere Metallionen, sein sollen, können für solche Filter nur solche Substanzen eingesetzt werden, bei denen eine Verunreinigung des Filtrats durch Metalle nicht zu erwarten ist. Deshalb wird in DE-41 19 288 vorgeschlagen, metallionenarme und vorzugsweise metallionenfreie Filterhilfsmittel wie feines hochdispersives Polymergranulat, mikronisierte Zellstoffe oder Mischungen dieser Stoffe als filtrationsaktive Komponente zu verwenden. Kieselgur oder Perlite, die wirksame Filterhilfsmittel sein könnten, scheiden wegen des Metallgehaltes aus.

WO 93/06911 beschreibt ein Verfahren zur Filtration von Blut mit Hilfe von pulverförmigem Crospovidone-Jod. Dieses wird bettförmig auf ein Filter aufgebracht Das Verfahren erfordert ein Crospovidone-Jod von weitgehend einheitlicher Korngröße von 40-150 µm. Dessen Herstellung ist technisch aufwendig. Außerdem verbleiben die abgetöteten Mikroorganismen im Filtrat und müssen daraus abgetrennt werden. Auch die Trennung der abfiltrierten zellulären Bestandteile des Blutes vom Crospovidone-Jod ist aufwendig und aufgrund der für die Anwendung erforderlichen engen Korngroößenverteilung für die Korngrößen großtechnisch schwierig zu realisieren.

In der WO 94/00161 ist die-Behandlung von Blut oder biologischem Gewebe mit Povidone-H₂O₂-Komplexen als biocider Substanzen beschrieben, wobei sowohl Povidone als auch Crospovidone als Basis für die Komplexe verwendet werden kann. Die Behandlung der biologischen Materialien kann in einer Vorrichtung erfolgen, in welcher Schichten verschiedener biocider Substanzen übereinander angeordnet sind.

Aufgabe der Erfindung war es, Tiefenfilter aus solchen Materialien zu finden, mit denen man die Reinigung und gleichzeitig das Abtöten von pathogenen Keimen und Inaktivieren von Viren in Flüssigkeiten, welche diese enthalten, vorteilhaft und ohne großen Aufwand durchführen kann. Verbunden mit dieser Aufgabe ist die Anwendung eines solchen Tiefenfilters zum Abtöten von pathogenen Keimen bei der Herstellung von reinem Wasser, pharmakologischen Lösungen, insbesondere Injektionslösungen und Blutplasmaprodukten.

Demgemäß wurden die eingangs definierten als biocide Tiefenfilter geeigneten Formkörper, Schichtenfilter aus einem oder mehreren solcher Formkörper sowie deren Verwendung für die Abtötung von Mikroorganismen und die Inaktivierung von Viren in diese enthaltenden Flüssigkeiten gefunden.

Die erfindungsgemäßen Förmkörper enthalten als biocide Substanz zur Abtötung von Mikroorganismen oder Inaktivierung von Viren Crospovidone-Jod.

Mit Crospovidone-Jod wird der erfindungsgemäß verwendete auf Basis von vernetztem PVP (PVPP, Crospovidone) Jodkomplex bezeichnet.

Im Gleichgewicht mit dem komplex gebundenen Jod steht das freie Jod. Dieser Anteil als wirksame Komponente wird in einem Konzentrationsbereich von 0,5 bis 6, vorzugsweise 1 bis 3 ppm stets aus dem Jodpool des Komplexes nachgebildet.

Das Jod kann in diesem Komplex in unterschiedlichen Mengen gebunden sein. Die handelsüblichen Produkte enthalten verfügbares Jod zwischen 9,0 und 12,0 Gew.-%, jedoch sind für die vorliegende Erfindung Produkte geeignet mit einem Gehalt an verfügbarem Jod zwischen 1 bis 20 Gew.-%. Die Korngröße des Crospovidone-Jods liegt zwischen 0,5 und 600 µm. Bevorzugt wird Crospovidone-Jod mit einer Korngrößenverteilung zwischen 0,5 und 250 µm verwendet. Die mittlere Korngröße liegt zwischen 30 und 40 µm. Die spezifische Oberfläche der Crospovidone-Jod-Partikel beträgt 0,8 bis 6, vorzugsweise 1,8 bis 2,5 m²/g [BET-Methode] , die Schüttdichte 0,1 bis 0,35, vorzugsweise 0,18 bis 0,25 g/ml.

Das Crospovidone-Jod ist in den als Tiefenfilter geeigneten Formkörpern in Mengen zwischen 0,5 und 75 Gew.-%, bevorzugt 10 bis 50 Gew.-%, besonders bevorzugt 20 bis 40 Gew.-%, bezogen auf das Gesamtgewicht des trockenen Formkörpers enthalten. Diese Mengen hängen einerseits von der im Crospovidone-Jod enthaltenen Menge an verfügbarem Jod und andererseits von der Art des zu reinigenden Materials ab. In einem Schichtenfilter selbst können Tiefenfilter mit verschiedenen Mengen an Crospovidone-Jod eingesetzt werden.

Die Partikel der biociden Substanz sind in die aus Fasermaterialien bestehenden Formkörper eingebettet. Die Formkörper bestehen aus Faserstoffen, wie sie für die Papier- und Filterherstellung bekannt sind, insbesondere Zellstoffasern. Ein geeigneter Faserstoff ist z. B. mikronisierter Zellstoff aus reiner Alpha-Zellulose mit einem Glührückstand von maximal 0,3 %. Die Faserlänge der verwendeten Alpha-Zellulose kann im Bereich von 10 bis 300 µm und vorzugsweise 18 bis 200 µm liegen, wobei eine durchschnittliche Faserdicke von 5 bis 30 µm zweckmäßig ist.

Zur Einstellung des erforderlichen Oberflächenpotentials können verschiedene Additive zugegeben werden. Zur Erzielung eines positiven Oberflächenpotentials kann man ein Polyamido-Aminepichlorhydrin-Harz, für ein negatives Oberflächenpotential modifizierte Galaktomannane oder Polyacrylamide zugeben. Durch Verwendung von anionischen Retentionshilfsmitteln kann ebenfalls ein negatives Oberflächenpotential erzielt werden.

Als Ersatz für die Zellulose oder zusätzlich zu der Zellulose ist es zweckmäßig, Polyolefinfasern, z. B. ein Polyethylenfibrid (PE-Fibrid) zuzusetzen. Ein solches PE-Fibrid kann beispielsweise eine Faserlänge von 50 bis 500 µm und eine Faserdicke von 0,1 bis 30 µm haben. Die Menge der zugesetzten Polyethylenfaser liegt im Bereich von 1 bis 60 Gew.-%, bezogen auf alle in der jeweiligen Filterschicht enthaltenen Bestandteile (Trockengewicht) und beträgt vorzugsweise 5 bis 25, und ganz besonders bevorzugt, ca. 10 Gew.-%.

Die Tiefenfilter können auch ausschließlich aus Polyethylenfibrid als Fasermaterialien bestehen.

Weiterhin kann es vorteilhaft sein, in die Filterschicht hochdispersives Polymergranulat einzuarbeiten. Besonders geeignet sind hierfür beispielsweise Harnstoff-Formaldehyd-Kondensate mit einer Primärteilchengröße von 0,1 bis 5 µm und einer spezifischen Oberfläche von 20 +/- 5 m²/g (BET-Methode) und Agglomeraten von Primärteilchen in der Größe zwischen 3 und 300 µm. Diese Polymergranulate können in Mengen von 1 bis 60 Gew.-% vorzugsweise 5 bis 20 Gew.-% verwendet werden. Auch Kieselgur oder Perlite können anstelle oder zusätzlich zu dem Polymergranulat innerhalb der angegebenen Menge von 1 bis 60 Gew.-% zugegeben werden, sofern sichergestellt ist, daß keine Metallionen daraus abgegeben werden oder abgegebene Metallionen an einer anderen Stelle des Filters festgehalten werden, oder abgegebene Metallionen im zu gewinnenden Filtrat nicht stören oder gegebenenfalls sogar erwünscht sind.

Bei der Herstellung der erfindungsgemäßen Tiefenfilter werden Verfahren und Vorrichtungen angewendet, wie sie für die Herstellung von üblichen Filtermaterialien verwendet werden. Für eine typische Verfahrensweise stellt man beispielsweise eine in der Papierindustrie übliche Pulpe in einem geeigneten Pulper her. Zur Herstellung dieser Pulpe verwendet man Faserstoffe, also beispielsweise Zellstoffe oder Polyolefinfasern, die zuvor in geeigneten Mahlaggregaten, z.B. Kegel-Refinern, fibrilliert wurden. Zu der so erhaltenen Suspension gibt man dann die für den jeweiligen Filteraufbau geeigneten Stoffe zu, also beispielsweise Harze, welche die Naßfestigkeit erhöhen und/oder das Zeta-Potential beeinflussen sowie das teilchenförmige Crospovidone-Jod in der geeigneten Menge etc.. Das Crospovidone-Jod kann aber auch schon vorher zu der Pulpe zugesetzt werden.

Die wäßrige Suspension mit allen in der Rezeptur vorgesehenen Inhaltsstoffen wird dann auf ein Sieb gegeben und dort schonend entwässert so daß man hochporöse Strukturen mit sehr hohem Trübaufnahmevermögen erhält. Anschließend erfolgt die Trocknung, üblicherweise in temperaturgesteuerten Mehretagentrocknern, wobei man die Trockentemperatur so regelt, daß die in der Mischung enthaltenen temperaturempfindlichen Komponenten nicht geschädigt werden. Hier soll die Trocknungstemperatur beispielsweise unterhalb der Erweichungstemperatur von im Filter enthaltenen Polyolefinfibriden liegen, üblicherweise im Bereich von 130°C.

Die Crospovidone-Jod enthaltenden Tiefenfilterpapiere haben im allgemeinen eine Dicke von 0,1 bis 10 mm. Das Flächengewicht der einzelnen Filterpapiere kann in weiten Bereichen variieren und liegt zwischen 20 g/m² und 2500 g/m². Der Aschegehalt der Filter variiert nach Art und Menge der im Filterpapier enthaltenen Stoffe. Er ist nicht kritisch, weil es nicht auf die im Filter zurückgehaltenen Substanzen ankommt, sondern auf die Reinheit des nach dem Filtrieren erhaltenen Filtrates.

Die Erfindung betrifft auch Schichtenfilter aus den erfindungsgemäßen Formkörpern. Schichtenfilter (Filterpressen) sind aus dem Stand der Technik bekannt. Sie enthalten ein oder mehrere Tiefenfilterschichten, durch welche die zu reinigende Flüssigkeit läuft, wobei die Schichtenfilter je nach Bedarf als Druck-, Saug- und hydrostatische Filter betrieben werden. Üblicherweise werden Tiefenfilter durchströmt, wobei die Schichten im Tiefenfilter unterschiedlich ausgebildet sein können, indem man z.B. die Art und die Dichte und/oder die Porengröße der Filterschichten variiert. In dem erfindungsgemäßen Schichtenfilter, der sich aus ein oder mehreren Tiefenfilterschichten bzw. Tiefenfilterpapieren aufbaut, ist in den Filterschichten Crospovidone-Jod, bevorzugt 10 bis 50 Gew.-%, besonders bevorzugt 20 bis 40 Gew.-% enthalten, das wie die löslichen Povidone-Jod-Komplexe (auf Basis von löslichem PVP) bakterizide, fungizide und viruzide Eigenschaften aufweist.

Bei der praktischen Anwendung des erfindungsgemäßen Tiefenfilters wird die zu reinigende Flüssigkeit, insbesondere Wasser, Injektionslösungen oder Blutplasmapräparate, auf das Filter aufgegeben. In der Regel erfolgt die Zugabe von oben, so daß am unteren Ende des Tiefenfilters die gereinigte Flüssigkeit gewonnen wird. Dabei arbeitet man zweckmäßigerweise mit einer Druckdifferenz über den gesamten Filter von oben nach unten von bis zu 5 bar. Die in die Poren der Schicht bzw. des Gefüges eindringenden Teilchen einschließlich Bakterien, Pilze oder Viren werden durch das in diesen Schichten vorliegende Crospovidone-Jod abgetötet bzw. inaktiviert, und die Rückstände setzten sich an der inneren Oberfläche und in den Hohlräumen der Schichten ab.

Für die Wirkungsweise und Effizienz der erf indungsgemäßen Tiefenfilter ist die durchschnittliche Verweilzeit der zu reinigenden Flüssigkeit beim Durchfließen des Tiefenfilters von erheblicher Bedeutung. Die Kontaktzeiten betragen 0,1 bis 5 min, vorzugsweise 1 bis 2 min. Diese Verweilzeit wird einerseits durch die Tiefe des zu durchströmenden Filterbettes als auch den inneren Aufbau der einzelnen Filterschichten beeinflußt. Es ist anzustreben, daß bei einer gegebenen Tiefe des Tiefenfilters eine optimale, zur Erzielung der bakteriziden, fungiziden und viruziden Eigenschaften ausreichende Verweilzeit sich einstellt. Der Kontakt der zu behandelnden Flüssigkeit mit den Crospovidone-Jodkomplexen im Gefüge des Tiefenfilters aufgrund der ständig wechselnden, meist stark turbulenten Strömungszustände in den Poren und Kanälen des Tiefenfilters ist sehr intensiv und dadurch ist die Wirksamkeit selbst bei sehr kurzen Kontaktzeiten höher als bei den Verfahren des vorerwähnten Standes der Technik. Durch die Kombination von Crospovidone-Jod enthalten Tiefenfilterschichten mit Crospovidone enthaltenden Tiefenfilterschichten (in dieser Reihenfolge) kann eventuell noch im Produkt befindliches Jod quantitativ entfernt werden. Diese Kombination kann je nach gewünschter Kontaktzeit in zwei verschiedenen Apparaten nacheinander oder durch Aufeinanderlegen der beiden Tiefenfilterschichten mit und ohne Spacer zwischen ihnen verwirkt werden.

Erfindungsgemäß mit Crospovidone-Jod ausgerüstete Tiefenfilterschichten bzw. Filterpapiere können auch in Filterpressen verwendet werden oder in Filterschichtenmodule oder in Filterkerzen in plisoierter Form eingebaut werden.

Versuche haben ergeben, daß man mit einem erfindungsgemäßen Tiefenfilter wirksam Wasser, biologische oder pharmazeutische oder chemische Flüssigkeiten, insbesondere aber Injektionslösungen und Blutplasmapräpate von pathogenen Keimen befreien kann, wobei auch die Rückstände(Debris) der abgetöteten Mikroorganismen im Filter zurückgehalten werden. Da die vorgenannten Flüssigkeiten ohnehin mindestens einem Filtriervorgang unterworfen werden müssen, kann durch die Verwendung eines Crospovidone-Jod enthaltenden Tiefenfilters die Reinigung in einfacher, zeitsparender und kostensparender Weise durchgeführt werden.

Die beanspruchten Tiefenfilter haben auch den Vorteil, daß sich im behandelten Filtrat kein Povidone befindet. Sofern partikuläre Bestandteile/Zellfraktionen im Medium enthalten sind, fallen diese bei der Filtration ohne Vermischung mit den Hilfsstoffen an. Für eine Weiterverarbeitung der Filtrationsrückstände entfallen somit alle Schritte, die der aufwendigen Abktrennung der Hilfsstoffe dienen.

### 1. Herstellungsbeispiel

Eine Tiefenfilterschicht zur Reinigung von biologischen Flüssigkeiten wie z.B. Blutplasmafraktionen wird wie folgt hergestellt:

Eine Zellstoffmischung aus hochgebleichten Sulfit-und Sulfatzellstoffen von Laub- und Nadelhölzern wurde in wäßriger Suspension (Pulpe) auf einen Mahlgrad von ca. 40o SR (Schopper Riegler) in Kegelrefinern gemahlen.

Zu 50 Gewicht steilen dieser Zellstoffmischung (bezogen auf Trockensubstanz) wurden 5 Gewichtsteile Polyethylenfibrid (PEFibrid, Type: ESS 21 der Fa. Schwarzwälder Textilwerke), 10 Gew.-% partikuläres Harnstoff-Formaldehyd-Kondensat (Agglomerate zwischen 6 und 60 mm) und 35 Gew.-% Crospovidone-Jod (Korngrößenverteilung 0,5 - 250 mm) zugesetzt. Die Naßverfestigung erfolgte durch Beigabe von ca. 0,5 Gew.-% Polyaminepichlorhydrin-Harz. Die homogene Suspension dieser Inhaltsstoffe wurde auf einem kontinuierlich laufenden Langsieb auf ca. 30-35% TS entwässert, wobei eine sehr poröse Struktur gebildet wird. Anschließend wird die nasse Filterschicht bei 130 bis 140°C in einem kontinuierlich betriebenen Etagentrockner bis zu einer Rest feuchte < 1% getrocknet und danach auf Format geschnitten. Das Ergebnis ist eine naßfeste, bakterizid, fungizid und viruzid wirkende Filterschicht zur Feinfiltration mit einer Porenverteilung zwischen 0,05 und 30 µm, wobei 90 % aller Poren zwischen 4 und 6 mm liegen (Meßprinzip: Coulter Porometer II) einer flächenbezogenen Masse von 1200 ± 50 g/m² und einer Dicke von 4 + 0,5 mm.

### Spezifikation des eingesetzten Crospovidone-Jod:

| Bezeichnung: | Crospovidone-Jod: |
|---|---|
| Stickstoffgehalt | 9,1 - 11,5% |
| Schwermetallgehalt | ≤ 10 ppm |
| Trocknungsverlust | ≤ 6% |
| Asche | < 0,3% |
| Verfügbares Jod | 9-12,0% |
| Freies Jod | 1-3 ppm |
| (10 Gew.-%ige Suspension in H₂O) | |

### 2. Anwendungsbeispiel Entkeimung von Bakterien-haltigen Medien

Die so hergestellten Tiefenfilterschichten wurden einer. Validierung mit dem Bakterienstamm Serratia marcescens unterzogen. Als Testmethode wurde die vom Arbeitskreis Technik/Analytik in der Europäischen Fachvereinigung Tiefenfiltration e.V. vorgeschlagene Methode (Vorschrift Nr. 4.2:Titerreduktion) verwendet ("Filtrieren und Separieren" (1994), Heft 5, S. 248-250). Diese Testmethode lehnt sich an die DIN 58355, Teil 3 "Bakterienrückhaltevermögen von Membranfiltern" an.

Bei den durchgeführten Tests wurde eine entsprechend dem Herstellungsbeispiel produzierte Filterschicht mit einer identischen Filterschicht verglichen, bei der der Crospovidone- Jod-Anteil durch die Zellstoffmischung, und einer ebenfalls identisch hergestellten Filterschicht, bei der der Crospovidone-Jod-Anteil durch eine Kieselgurmischung aus Feinguren mit einer Permeabilität von 0,1 Darcy (Methode 1.3 ''Permeabilität", Arbeitskreis Technik/Analytik i .d. Europäischen Fachvereinigung Tiefenfiltration e.V.) ersetzt wurde.

Diese Ergebnisse werden in LRV-Werten angegeben.
(LRV = logarithmic reduction value)

### Ergebnisse: LVR

| | | |
|---|---|---|
| 1. | Tiefenfilter mit 35% Crospovidone-Jod | 10 |
| 2. | Tiefenfilter ohne Crospovidone - Jod | 2 |
| 3. | Tiefenfilter mit 35% Kieselgurmischung | 5 |

Das heißt, daß die Tiefenfilterschicht mit 35% Crospovidone-Jod (1) bei einer Belastung von 108 Keimen in 100 ml Unfiltrat pro 20 cm² Filterfläche (Serratia marcescens) noch ein steriles Filtrat liefert. Bei gleicher Belastung liefert die Zellstoffschicht (2) ein Filtrat mit 10 Keimen in 100 ml und die Feinklärschicht (3) mit 35% Kieselgur ein Filtrat mit 105 Keimen pro 100 ml. D.h. die Crospovidone-Jod enthaltende Tiefenfilterschicht hat eine um 5 Zehnerpotenzen bessere Entkeimungsleistung verglichen mit üblichen kieselgurhaltigen Filterschichten.

### Inaktivierung von Viren

Dien Inaktivierung von Virussuspensionen bei Filtration durch erfindungsgemäße Tiefenfilter wurde wie im folgenden geschildert quantitativ bestimmt.

Die Infektiosität der Virensuspensionen wurde durch Bestimmung des zytopathischen Effekts der Viren auf Indikatorzellen in Zellkulturen vor und nach der Filtration gemessen. Die Titerrreduktion gibt den Grad an Virusinaktivierung an.

### Materialien:

a) Testviren
   - Herpes-simplex-Virus Typ 1: HSV-A strain Mc Intyre
   - HIV-1: ursprünglich HTLV-IIIB genanntes Isolat (Gallo-Popovic)
b) Zellkulturen zu Virusstockherstellung und Titration:
   - menschliche Fibroblasten α 1
   - African-green-monkey-kidney-Zellen (GMK)
   - MT-2- bzw. MT-4-T-Lymphomzellinien
c) Zellkulturmedien
   - Eagle's MEM (Seromed/Biochrom) mit fetalem Kälberserum (FKS; 5 %), Penicillin (100 E/ml) und Streptomycin (0,1 mg/ml).
   - RPMI-Medium (Seromed, Biochrom), 10 % FKS, Antibiotika: Penicillin (40 E/ml), Streptomycin (0,04 mg/ml)
d) Filtrationsmedien
   - Eagle's MEM ohne bzw. mit 10 % FKS, RPMI, ohne bzw. mit 10 % FKS
e) Geräte
   Filterhalter und peristaltische Pumpe der Firma Schenk Filterbau
   Filter hergestellt aus Zellstoff und 20 Gew.-% bzw. 40 Gew.-% Crospovidone-Jod sowie zum Vergleich ohne Crosposdone-Jod.

### Versuche:

Die eventuelle Toxizität des Filtrates für die verwendeten Zellinien wurde mit den 40 % und 20 % Crospovidone-Jod enthaltenden Zellstoffschichten getestet.
Die Filterschicht wurde mit dem Filtrationsmedium Eagle's MEM ohne FKS gespült. Die ersten filtrierten 100 ml (Filtrat A) und die nächsten 100 ml (Filtrat B) wurden aufgefangen. Die Kulturen wurden an den folgenden fünf Tagen auf Veränderungen des zellwachstums und der Zellmorphologie hin überprüft.

Mit den Filtraten A und B aus der 20 % Filterschicht wurden kein toxischer Effekt auf die Zellen beobachtet. Mit dem Filtrat A aus der 40 % Filterschicht wurde eine toxische Wirkung auf α1- und GMK-Zellen in der ersten Stufe der Verdünnungsreihe beobachtet. Mit Filtrat B wurde keine toxische Wirkung festgestellt.
MT-2-Zellen wurden durch Filtrat aus Filtern mit 40 % Crospovidone-Jod ohne FKS bei der ersten Titrationsstufe im Wachstum gehemmt, aber nicht geschädigt. Das entsprechende Filtrat mit FKS veränderte Zelleachstum und Zellmorphologie nicht.
Somit wurde beim Inaktivierungsversuch das Testvirus erst nach Passage von 200- bzw. 250 ml Medium durch die Filter aufgetragen.

Um auszuschließen, daß das Virus erst nach der Filtration im Filtrat und nicht, wie angestrebt, in der Filterschicht inaktiviert wird, wurden Filtrate A und B mit einer HSV-1-Stammsuspension versetzt und dann titriert. Mit Filtrat A war der Titer um eine halbe log₁₀ Stufe reduziert, während mit Filtrat B der gleiche Titer wie mit der entsprechend verdünnten Stammsuspension gemessen wurde. Somit war eine Inaktivierung durch das Filtrat ausgeschlossen.

Es wurde weiterhin geprüft, ob Viren an die Filterschicht ohne Crospovidone-Jod adsorbiert wird. Dazu wurden die HSV-Stocksuspension vor und nach Filtration auf einer Filterschicht, die kein Crospovidone-Jod enthielt, titriert.

### Hauptversuche:

Die Filterschicht wird mit 200 bis 250 ml Eagle's MEM (bei Versuchen mit HSV-1) oder RPMI-Medium (HIV-1) vorgespült. Die Filtrationsgeschwindigkeit beträgt 16 ml/min. Ohne die Filtration zu unterbrechen wird 1 ml der Virussuspension in 20 Sekunden in den Flüssigkeitszulauf des Filters injiziert. Danach werden 100 ml Filtrat steril aufgefangen (1. Filtrat). Der Inkektions- und Filtrationsovorgang wird am selben Filter zweimal wiederholt (2. und 3. Filtrat). Die ungefilterte Virusstocksuspension wird mit dem Filtrat parallel titriert:
in 24-Loch-Platten in log₁₀-Verdünnungsstufen (4fach für HSV); HIV wurde in 96-Loch-Platten in dreifach-Verdünnungsstufen und drei Parallelansätzen titriert. Der von den Viren verursachte typisch zytopathogene Effekt bzw. die Synzytienbilding bei HIV wird über fünf Tage beobachtet.

Die Zellkultur-infizierenden Einheiten-50 (TCID₅₀ [tissue culture Infactious dose 50%]) wurden nach einem statistischen Verfahren von Spearman und Kärber errechnet. Die Reduktion der Infektiosität wird in Form der log₁₀ TCID₅₀/ml angegeben.

Die Ergebnisse der Inaktivierungsversuche mit HSV-1 sind in der nachstehenden Tabelle 1 aufgeführt.

Die Filtrate wurden wie angegeben titriert. Vor der Filtration betrugen die Virusdosen 10^{8,5} (Versuch 1) bzw. 10⁷ (Versuch 2) TCID₅₀/ml. Versuch 1 wurde in Anwesenheit von FKS durchgeführt. Die Titerreduktion ist in Logarithmen zur Basis 10 angegeben.

**Tabelle 1**

| Versuch Nr. | 1 | 2 |
|---|---|---|
| Konzentration Crospovidone-Jod in Gew.-% | 20 | 20 |
| FKS | + | - |
| 1 | ≥ 7 | ≥ 5,5 |
| 2 | ≥ 7 | ≥ 5,5 |
| 3 | 6,5 | ≥ 5,5 |

Die Ergebnisse der Inaktivierungsversuche mit HIV-1 sind in Tabelle 2 aufgeführt.

Angegeben ist die Anzahl der infektiösen Einheiten im Filtrat (TCID₅₀).

**Tabelle 2**

| Versuch Nr. | 3 | 4 | 5 |
|---|---|---|---|
| Konzentration Crospovidone-Jod in Gew.-% | 0 | 40 | 40 |
| FKS | + | + | - |
| Filtrat 1 | 120 000 | ≤ 1 | ≤ 1 |
| Filtrat 2 | n.d. | ≤ 1 | ≤ 1 |
| Filtrat 3 | n.d. | ≤ 1 | ≤ 1 |
| n.d. = Nicht durchgeführt | | | |

Damit wurden durch die. Filtration 10⁵ infektiöse Einheiten inaktiviert.

## Patentansprüche

1. Als biocide Tiefenfilter geeignete Formkörper,
- bestehend aus Fasermaterialien, und aus
- Crospovidone-Iod als biocider Substanz, ohne Crospovidone - Wasserstoffperoxid
wobei die biocide Substanz partikulär in die Formkörper eingebettet ist.

2. Formkörper nach Anspruch 1 enthaltend Crospovidone-Jod mit 1 bis 20 Gew.-% verfügbarem Jod.

3. Formkörper nach Anspruch 2, enthaltend 0,5 bis 75 Gew.-%, bezogen auf das Gesamtgewicht des Formkörpers, an Crospovidone-Jod.

4. Formkörper nach Anspruch 2 oder 3 mit einer Korngrößenverteilung der Crospovidone-Jod-Partikel im Bereich von 0,5 bis 600 µm.

5. Formkörper nach einem der Ansprüche 2 bis 4, mit einer mittleren Korngröße der Crospovidone-Jod-Partikel zwischen 30 und 40 µm.

6. Formkörper nach einem der Ansprüche 1 bis 5, enthaltend als Faserkomponenten Zellstoff und/oder Kunststoffasern.

7. Formkörper nach einem der Ansprüche 1 bis 6, enthaltend Faserkomponenten aus fibrillierten Zellstoffasern und/oder Polyolefinfasern.

8. Formkörper nach einem der Ansprüche 1 bis 7, enthaltend hochdisperses Polymergranulat und/oder Kieselgur und/oder Perlit in einer Menge von 1 bis 60 Gew.-% bezogen auf das Trockengewicht.

9. Formkörper nach Anspruch 8, enthaltend als hochdisperses Polymergranulat ein Harnstoff-Formaldehyd-Kondensationsprodukt.

10. Formkörper nach einem der Ansprüche 1 bis 9 enthaltend durch Trockenmahlung hergestelltes Zellstoffpulver mit einer Partikelgröße von 1 bis 100 µm in einer Menge von 1 bis 60 Gew.-% bezogen auf das Gesamtgewicht (trocken).

11. Formkörper nach einem der Ansprüche 1 bis 9, wobei die Tiefenfilterschicht eine Dicke von 0,1 bis 10 mm und ein Flächgengewicht von 20 bis 3500 g/m² aufweist.

12. Schichtenfilter, enthaltend als Tiefenfilterschicht einen oder mehrere Formkörper nach einem der Ansprüche 1 bis 10.

13. Schichtenfilter nach Anspruch 12, enthaltend vor oder hinter den Crospovidone-Jod enthaltenden Filterschichten weiter Filterschichten, welche kein Crospovidone-Jod enthalten.

14. Schichtenfilter nach Anspruch 12 oder 13, enthaltend Tiefenfilterschichten in Form von Schichtenmodulen.

15. Schichtenfilter nach einem der Ansprüche 12 bis 14, enthaltend Tiefenfilterpapiere in Form von plissierten Filterkerzen.

16. Verwendung eines Tiefenfilters gemäß einem der Ansprüche 1 bis 10 zum Abtoten von Mikroorganismen und Inaktivieren von Viren in solche enthaltenden Flüssigkeiten.

17. Verwendung nach Anspruch 12, dadurch gekennzeichnet, daß es sich um Wasser, eine pharmazeutische oder biologische Flüssigkeit handelt.

18. Verwendung nach Anspruch 13, dadurch gekennzeichnet, daß die zu reinigende Flüssigkeit eine Injektionslösung ist oder ein Blutplasmapräparat enthält.

## Claims

1. A shaped article which is suitable as biocidal depth-type filter,
- and which consists of fibrous materials, and of
- crospovidone-iodine as biocidal substance, without crospovidone-hydrogen peroxide
where the biocidal substance is embedded as particles in the shaped article.

2. A shaped article as claimed in claim 1, containing crospovidone-iodine with 1 to 20% by weight of available iodine.

3. A shaped article as claimed in claim 2, containing 0.5 to 75% by weight, based on the total weight of the shaped article, of crospovidone-iodine.

4. A shaped article as claimed in claim 2 or 3, with a particle size distribution of the crospovidone-iodine particles in the range from 0.5 to 600 µm.

5. A shaped article as claimed in any of claims 2 to 4, with an average particle size of the crospovidone-iodine particles of from 30 to 40 µm.

6. A shaped article as claimed in any of claims 1 to 5, containing as fibrous components cellulose and/or synthetic fibers.

7. A shaped article as claimed in any of claims 1 to 6, containing fibrous components of fibrillated cellulose fibers and/or polyolefin fibers.

8. A shaped article as claimed in any of claims 1 to 7, containing highly disperse polymer granules and/or kieselguhr and/or perlite in an amount of from 1 to 60% by weight based on dry weight.

9. A shaped article as claimed in claim 8, containing a urea/formaldehyde condensation product as highly disperse polymer granules.

10. A shaped article as claimed in any of claims 1 to 9, containing cellulose powder which has been produced by dry milling and has a particle size of from 1 to 100 µm in an amount of from 1 to 60% by weight based on the total weight (dry).

11. A shaped article as claimed in any of claims 1 to 9, where the depth-type filter layer has a thickness of from 0.1 to 10 mm and an area weight of from 20 to 3,500 g/m².

12. A layer filter containing as depth-type filter layer one or more shaped articles as claimed in any of claims 1 to 10.

13. A layer filter as claimed in claim 12, containing, before or after the crospovidone-iodine-containing filter layers, other filter layers which contain no crospovidone-iodine.

14. A layer filter as claimed in claim 12 or 13, containing depth-type filter layers in the form of layer modules.

15. A layer filter as claimed in any of claims 12 to 14, containing depth-type filter papers in the form of pleated filter candles.

16. The use of a depth-type filter as claimed in any of claims 1 to 10 for killing microorganisms and inactivating viruses in liquids containing such.

17. The use as claimed in claim 12, which applies to water, a pharmaceutical or biological liquid.

18. The use as claimed in claim 13, wherein the liquid to be purified is an injection solution or contains a blood plasma product.

## Revendications

1. Corps façonné convenant comme filtre à lit profond biocide,
- constitué de matériaux fibreux, et
- d'un composé iode-crospovidone, à titre de substance biocide, sans composé peroxyde d' hydrogène-crospovidone,
la substance biocide étant noyée sous forme de particules dans le corps façonné.

2. Corps façonné selon la revendication 1, contenant un composé iode-crospovidone contenant de l'iode disponible en proportion de 1 à 20 % en poids.

3. Corps façonné selon la revendication 2, contenant de 0,5 à 75 % en poids d'iode-crospovidone, en se référant au poids total du corps façonné.

4. Corps façonné selon la revendication 2 ou 3, présentant une distribution de taille de grains des particules d'iode-crospovidone située dans la plage allant de 0,5 à 600 µm.

5. Corps façonné selon l'une des revendications 2 à 4, comportant une taille de grains moyenne des particules d'iode-crospovidone comprise entre 30 et 40 µm.

6. Corps façonné selon l'une des revendications 1 à 5, contenant comme composant fibreux, des fibres cellulose et/ou de matériau plastique.

7. Corps façonné selon l'une des revendications 1 à 6, contenant des composants fibreux constitués de fibres de cellulose fibrillées et/ou des fibres de polyoléfine.

8. Corps façonné selon l'une des revendications 1 à 7, contenant, à l'état fortement dispersé, un granulat de polymère et/ou de la diatomite et/ou de la perlite en une quantité comprise dans la plage allant de 1 à 60 % en poids par rapport au poids à sec.

9. Corps façonné selon la revendication 8, contenant, comme granulat fortement dispersé, un produit de condensation urée-formaldéhyde.

10. Corps façonné selon l'une des revendications 1 à 9, contenant une poudre de cellulose, fabriquée par broyage à sec, ayant une taille de particules comprise dans la plage allant de 1 à 100 µm en une quantité allant de 1 à 60 % en poids par rapport au poids total (à sec).

11. Corps façonné selon l'une des revendications 1 à 9, la couche du filtre à lit profond ayant une épaisseur de 0,1 à 10 mm et un poids par unité de surface de 20 à 3500 g/m².

12. Filtre à couches, contenant comme couche de filtre à lit profond un ou plusieurs corps façonné(s) selon l'une des revendications 1 à 10.

13. Filtre à couches selon la revendication 12, contenant, devant ou derrière les couches de filtre contenant de l'iode-crospovidone, d'autres couches de filtre qui ne contiennent pas d'iode-crospovidone.

14. Filtre à couches selon la revendication 12 ou 13, contenant des couches de filtre à lit profond se présentant sous la forme de modules à couches.

15. Filtre à couches selon l'une des revendications 12 à 14, contenant des papiers pour filtre à lit profond se présentant sous la forme de bougies filtrantes plissées.

16. Utilisation d'un filtre à lit profond selon l'une des revendications 1 à 10, pour la destruction des micro-organismes et l'inactivation des virus dans des liquides contenant de tels virus.

17. Utilisation selon la revendication 12, caractérisée par le fait qu'il s'agit d'eau, d'un liquide pharmaceutique ou biologique.

18. Utilisation selon la revendication 13, caractérisée par le fait que le liquide à épurer contient une solution d'injection ou bien une préparation de plasma sanguin.
